(19) Europäisches Patentamt
European Patent Office
Office européen des brevets

(11) EP 0 975 582 B1

(12) **EUROPÄISCHE PATENTSCHRIFT**

(45) Veröffentlichungstag und Bekanntmachung des Hinweises auf die Patenterteilung:
**06.11.2002 Patentblatt 2002/45**

(21) Anmeldenummer: **98917048.5**

(22) Anmeldetag: **23.03.1998**

(51) Int Cl.[7]: **C07C 229/24**, C07C 227/08

(86) Internationale Anmeldenummer:
**PCT/EP98/01670**

(87) Internationale Veröffentlichungsnummer:
**WO 98/045251 (15.10.1998 Gazette 1998/41)**

(54) **HERSTELLUNG UND VERWENDUNG VON IMINODIBERNSTEINSÄURESALZEN**

PREPARATION AND USE OF IMINODISUCCINIC ACID SALTS

PREPARATION ET UTILISATION DE SELS D'ACIDE IMINODISUCCINIQUE

(84) Benannte Vertragsstaaten:
**AT BE CH DE DK ES FI FR GB IT LI NL PT SE**

(30) Priorität: **04.04.1997 DE 19713911**

(43) Veröffentlichungstag der Anmeldung:
**02.02.2000 Patentblatt 2000/05**

(60) Teilanmeldung:
**02010124.2 / 1 247 800**

(73) Patentinhaber: **BAYER AG**
**51368 Leverkusen (DE)**

(72) Erfinder:
- **GROTH, Torsten**
  **D-51519 Odenthal (DE)**
- **JOENTGEN, Winfried**
  **D-51067 Köln (DE)**
- **WAGNER, Paul**
  **D-40597 Düsseldorf (DE)**
- **DÖBERT, Frank**
  **D-51065 Köln (DE)**
- **WENDEROTH, Eckhard**
  **D-51381 Leverkusen (DE)**
- **ROICK, Thomas**
  **D-51375 Leverkusen (DE)**

(56) Entgegenhaltungen:
**EP-A- 0 831 165**

- **DATABASE WPI Section Ch, Week 9507 Derwent Publications Ltd., London, GB; Class D25, AN 95-048800 XP002069425 & JP 06 329 606 A (NIPPON SHOKUBAI CO LTD) in der Anmeldung erwähnt**
- **DATABASE WPI Section Ch, Week 9616 Derwent Publications Ltd., London, GB; Class D25, AN 96-157376 XP002069426 & JP 08 041 490 A (NIPPON SHOKUBAI CO LTD)**
- **DATABASE WPI Section Ch, Week 9612 Derwent Publications Ltd., London, GB; Class D25, AN 96-112673 XP002069427 & JP 08 012 631 A (NITTO CHEM IND CO LTD)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 975 582 B1

Printed by Jouve, 75001 PARIS (FR)

## Beschreibung

Die Erfindung betrifft ein Verfahren zur Herstellung von Iminodibernsteinsäurealkalisalzen durch Umsetzung von Maleinsäure und Ammoniak in wäßrigem Medium in Gegenwart von Alkalimetallhydroxiden und deren Aufarbeitung. Die daraus resultierenden Produkte können als Komplexiermittel für Erdalkali- und Schwermetallionen in den Bereichen Wasch- und Reinigungsmittel, Pharma, Kosmetik, Landwirtschaft, Galvanik, Baustoffe, Textil und Papier eingesetzt werden. In diesen Bereichen ist die Verwendung als Wasserenthärter, Bleichmittelstabilisator, Spurennährstoffdünger und Abbindeverzögerer besonders hervorzuheben. Die Erfindung betrifft weiterhin die Verwendung von Iminodibernsteinsäurealkatisalzen bei der Papierherstellung.

Komplexiermittel werden seit Jahren in großen Mengen eingesetzt. Viele bisher gebräuchliche Komplexiermittel, wie Ethylendiamintetraessigsäure (EDTA), Diethylentriaminpentaessigsäure (DTPA), Nitrilotriessigsäure (NTA) und verschiedene Phosphonate sind nicht oder nur bedingt biologisch abbaubar, remobilisieren Schwermetalle in Oberflächengewässern und können sogar bis in die Trinkwasseraufbereitung gelangen, da sie weder in Klärschlämmen noch in Böden adsorbiert werden. Phosphate sind Komplexiermittel, die zur Eutrophierung von Gewässern beitragen. Zusammengefaßt handelt es sich dabei um ökotoxikologische Eigenschaften, die in der heutigen Zeit als nachteilig empfunden werden.

Daher ist es eine wichtige Aufgabe, Komplexiermittel zu entwickeln, die die bisherigen ökotoxikologischen Nachteile nicht aufweisen. Iminodibernsteinsäure ist nun ein Komplexiermittel, das eine leichte biologische Abbaubarkeit zeigt und damit gegenüber den bisherigen Komplexiermitteln einen ökotoxikologischen Vorteil besitzt.

Zukünftig werden aber nicht nur die Produkteigenschaften von Chemikalien, die nach dem Gebrauch überwiegend in die Umwelt gelangen, unter den geschilderten Aspekten kritisch geprüft, sondern auch die Herstellungsverfahren. So war es überraschend, daß für eine industriell zur Zeit noch nicht verfügbare Chemikalie, die umweltgerechte Eigenschaften besitzt, auch ein umweltgerechtes Herstellungverfahren gefunden werden konnte.

Für Iminodibernsteinsäure sind auf der Basis von Maleinsäureanhydrid oder Maleinsäure und Ammoniak bisher folgende Herstellungsmöglichkeiten bekannt: In GB 1 306 331 wird die Herstellung von Iminodibernsteinsäure aus Maleinsäure und Ammoniak im Molverhältnis von 2:3 bis 2:5 bei Temperaturen von 60 bis 155°C beschrieben. Zur Aufarbeitung werden entweder Salzsäure oder Natronlauge hinzugegeben. In SU 0 639 863 wird Iminodibernsteinsäure in Gegenwart von Alkalimetallhydroxiden aus Maleinsäure und Ammoniak bei einem Molverhältnis von 2: 0,8 bis 2:1 und Temperaturen von 110 bis 130°C hergestellt. In JP 6/329 606 wird ein dreistufiges Verfahren zur Herstellung von Iminodibernsteinsäure beschrieben. Zunächst reagiert ein Maleinsäurederivat mit Ammoniak in wäßrigem Medium. Danach erfolgt die Zugabe von Alkali- oder Erdalkalimetallhydroxiden. In der dritten Verfahrensstufe schließt sich ein sogenannter Reifungsprozeß an. In JP 6/329 607 wird ebenfalls ein dreistufiges Verfahren zur Herstellung von Iminodibernsteinsäure beschrieben. In der ersten Stufe wird wiederum zunächst ein Maleinsäurederivat mit Ammoniak in wäßrigem Medium umgesetzt. Danach erfolgt in der zweiten Stufe der Zusatz von Alkali- oder Erdalkalimetallhydroxiden. In der dritten Stufe wird nach Zusatz von weiterem Maleinsäurederivat die Reaktion fortgesetzt. In dieser Patentanmeldung wird ausdrücklich gesagt, daß als Maleinsäurederivate Maleinsäureanhydrid, Maleinsäure oder das Maleinsäureammoniumsalz eingesetzt werden. Mit den Metallsalzen der Maleinsäure soll die gewünschte Reaktion kaum stattfinden, so daß man das Ziel nicht erreichen kann.

Um so erstaunlicher ist es, daß erfindungsgemäß gerade in Gegenwart von Alkalimetallhydroxiden Maleinsäure und Ammoniak in vorteilhafter Weise zu Iminodibernsteinsäure in hohen Ausbeuten umgesetzt werden konnten.

Die Erfindung betrifft daher ein Verfahren zur Herstellung von Iminodibernsteinsäurealkalisalzen, das dadurch gekennzeichnet ist, daß Maleinsäureanhydrid (MSA), Alkalimetallhydroxid (MeOH), Ammoniak ($NH_3$) und Wasser im Molverhältnis von MSA : MeOH : $NH_3$ : $H_2O$ = 2:0,1-4:1,1-6:5-30 bei Temperaturen von 70-170°C, unter Drücken von 1-80 bar und Reaktionszeiten von 0,1-100 h umgesetzt werden, aus dem Umsetzungsgemisch unter Zusatz von Wasser und 0-4 Mol MeOH pro 2 Mol ursprünglich eingesetztem MSA Ammoniak und Wasser bei Temperaturen von 50-170°C unter Drücken von 0,1-50 bar innerhalb von 0,1-50 h abdestilliert werden und nach der Destillation Wasser in einer solchen Menge zugesetzt wird, daß die entstehende Lösung einen Feststoffgehalt von 5-60 %, bezogen auf das Gesamtgewicht der Lösung, enthält.

Im erfindungsgemäßen Verfahren werden in einem Reaktor Wasser, Alkalimetallhydroxid (MeOH), Maleinsäureanhydrid (MSA) und Ammoniak ($NH_3$) eindosiert und das entstandene Maleinsäuresalz bei den genannten Reaktionstemperaturen (T) und Reaktionszeiten (t) umgesetzt. Anschließend werden unter Zusatz von Wasser und gegebenenfalls weiterem MeOH Ammoniak im Gemisch mit Wasser abdestilliert. Nach dieser Destillation wird das Produkt durch Zugabe von Wasser auf eine zweckmäßige Konzentration eingestellt. Diese Produktlösung kann gegebenenfalls einer Klärfiltration unterzogen werden. Me bedeutet hierbei Li, Na oder K, bevorzugt Na oder K, besonders bevorzugt Na.

Das erfindungsgemäße Verfahren hat den Vorteil, daß es sowohl diskontinuierlich als auch kontinuierlich durchgeführt und dabei ein hohes Maß an Wirtschaftlichkeit erreicht werden kann. Dieser Sachverhalt ist von großer Bedeutung, da auch umweltfreundliche Produkte trotz aller Vorteile nur konkurrenzfähig sind, wenn sie unter entsprechend ökonomischen Bedingungen hergestellt werden können. Das erfindungsgemäße Verfahren erzeugt keinen Abfall, da nach der Destillation des Ammoniaks, der recycliert und weiter verwertet und gegebenenfalls wieder eingesetzt werden kann, das verbleibende Produkt vollständig verwendet wird. Dieses Produkt ist darüber hinaus nach OECD 301 E leicht biologisch abbaubar. Im Verfahren und Produkt werden Ökonomie und Ökologie in bisher nicht bekannter Weise miteinander vereint.

Im erfindungsgemäßen Verfahren werden zunächst MSA, Wasser und Alkalimetallhydroxid im Molverhältnis von MSA : MeOH : Wasser = 2:0,1-4:5-30 miteinander vermischt, wobei unterschiedliche Dosiervarianten ausgeführt werden können. So kann MSA zunächst mit Wasser über die Stufe der Maleinsäure oder alternativ direkt mit wäßriger Alkalimetallhydroxidlösung zu den entsprechenden Maleinsäuresalzen umgewandelt werden. Aus technischen und chemischen Gründen hat sich die zweite Dosiervariante als vorteilhaft erwiesen. Mit ihr ist es möglich, auf einfache Weise besonders konzentrierte und an Nebenkomponenten arme Maleinsäuresalzlösungen herzustellen. Diese leicht gelblichen Lösungen enthalten die möglichen Nebenkomponenten Fumarsäure und Äpfelsäure nur in geringen Mengen. So wird das Maleinsäuresalz mit Ausbeuten von >92 %, bevorzugt >95 %, besonders bevorzugt >98 % der theoretischen Menge im weiteren Prozeß verwendet.

Im Hinblick auf eine kontinuierliche Prozeßführung hat sich die kontinuierliche und simultane Dosierung von MSA und Alkalimetallhydroxidlösungen in eine vorgelegte, Maleinsäuresalzlösung als besonders vorteilhaft erwiesen. Auf diese Weise können sogar sehr reine und auch farblose Lösungen mit ebenso hohen Ausbeuten erhalten werden.

In bevorzugter Weise werden MSA und MeOH in einem Molverhältnis von 2:0,5-3,9, besonders bevorzugt 2:0,9-3,5, ganz besonders bevorzugt 2:1,5-3,1, eingesetzt.

In bevorzugter Weise werden MSA und NH3 in einem Molverhältnis von 2:1,2-5,5, besonders bevorzugt 2:1,5-4,5, ganz besonders bevorzugt 2:1,9-3,5, eingesetzt.

In bevorzugter Weise werden MSA und $H_2O$ in einem Molverhältnis von 2:5,5-25, besonders bevorzugt 2:6-20, ganz besonders bevorzugt 2:6,5-15, eingesetzt.

Die Herstellung des Maleinsäuresalzes aus MSA, MeOH und Wasser erfolgt bei Temperaturen von mindestens 60°C, beispielsweise bei 60-130°C, bevorzugt bei 70-120°C, besonders bevorzugt bei 80-115°C, bei denen eine schnelle und vollständige Reaktion des MSA gewährleistet ist und bei denen eine Rührfähigkeit und Pumpfähigkeit der Mischung aufrecht erhalten werden kann. So kann das Maleinsäuresalz als Suspension oder Lösung, bevorzugt als Lösung vorliegen, die außerdem über mehrere Stunden gerührt werden kann, ohne daß nennenswerte Ausbeuteverluste auftreten würden.

Zu den aus MSA, MeOH und Wasser gebildeten Maleinsäuresalz-Suspensionen oder - Lösungen wird Ammoniak in einem Molverhältnis von MSA : Ammoniak = 2:1,1-6, bevorzugt 2:1,2-5,5, besonders bevorzugt 2:1,5-4,5 dosiert. Die Zugabe kann gleichermaßen sowohl in diskontinuierlicher als auch in kontinuierlicher Prozeßführung erfolgen.

Die aus MSA, MeOH, Ammoniak und Wasser gebildeten Maleinsäuresalzlösungen werden bei Temperaturen von 70-170°C, bevorzugt bei 80-160°C, besonders bevorzugt bei 85-150°C, ganz besonders bevorzugt 90-145°C und Reaktionszeiten von 0,1-100 h, bevorzugt 0,2-50 h, besonders bevorzugt 0,3-25 h, ganz besonders bevorzugt 0,5-20 h umgesetzt. Die Reaktion kann sowohl in Diskonti- als auch Konti-Reaktoren durchgeführt werden.

Die Reaktion wird in der Regel unter dem sich automatisch einstellenden Druck durchgeführt. Dabei können Drücke von bis zu 50 bar, bevorzugt bis zu 30 bar, besonders bevorzugt bis zu 20 bar auftreten. Eine Überlagerung mit Inertgasen, besonders in Diskonti-Reaktoren, kann dabei zusätzlich vorgenommen werden, wobei Drücke bis zu 80 bar zulässig sind.

Durch die Reaktionsbedingungen wird ein Maleinsäure-Umsatz von >93 %, bevorzugt >95 %, besonders bevorzugt >98 % des theoretischen Umsatzes erreicht.

Nach der Reaktion werden aus dem Umsetzungsgemisch unter Zusatz von Wasser und 0-4 Mol, bevorzugt 0,5-3,5 Mol, besonders bevorzugt 0,7-3,0 Mol, ganz besonders bevorzugt 0,9-2,5 Mol MeOH pro 2 Mol ursprünglich eingesetztem MSA Ammoniak und Wasser abdestilliert. Der Zusatz von Wasser und MeOH kann vor oder während der Destillation sowohl im Diskonti- als auch Konti-Prozeß erfolgen. Die Menge des zugesetzten Wassers wird unter Berücksichtigung des unvermeidlich mit dem $NH_3$ abdestillierenden Wassers so bemessen, daß im verbleibenden Aufarbeitungsgemisch ein Feststoffgehalt von 75 Gew.%, bevorzugt von 70 Gew.-%, besonders bevorzugt von 65 Gew.-%, bezogen auf das Gesamtgewicht des Ansatzes, nicht überschritten wird.

Die Destillation erfolgt bei Temperaturen von 50-170°C, bevorzugt bei 60-150°C, besonders bevorzugt bei 70-140°C, ganz besonders bevorzugt 80-135°C und Drücken von 0,1-50 bar, bevorzugt 0,5-20 bar innerhalb von 0,1-50 h, bevorzugt 0,3-30 h, besonders bevorzugt 0,5-25 h, ganz besonders bevorzugt 0,9-20 h. Dadurch wird eine weitestgehende Abtrennung von nicht umgesetztem und hydrolysierbarem Ammoniak (z.B. durch Kondensation gebildeter Amidstickstoff RCONH2 + MeOH ----> RCOOMe + NH3 mit R = Kohlenwasserstoffrest der zugrundeliegenden Carbonsäure) aus dem Umsetzungsgemisch erreicht, der anschließend aufbereitet werden und für eine Weiterverwertung genutzt werden kann. Dadurch wird außerdem eine Minimierung des Stickstoffgehaltes im Produkt erreicht und so bei der Verwendung ein unnötig hoher Stickstoffeintrag in Gewässer vermieden. Der Gehalt der nach der Reaktion noch

verbliebenen freien Maleinsäure wird ebenfalls weiter veringert.

Nach der Destillation wird Wasser in einer solchen Menge zugesetzt, daß die entstehende Produktlösung einen Feststoffgehalt, gerechnet als Summe aller Alkalisalze, von 5-60 Gew.-%, bevorzugt 10-58 Gew-.%, besonders bevorzugt 15-55 Gew.-% aufweist. Im Anschluß daran kann im Bedarfsfall eine Klärfiltration durchgeführt werden. Derartige Lösungen sind nahezu geruchsneutral und lagerstabil.

Nach Reaktion und Aufarbeitung werden Iminodibernsteinsäure und ihre Salze (Formel 1) in Ausbeuten von >65 %, bevorzugt >70 %, besonders bevorzugt >74% der theoretischen Ausbeute erhalten. Die Summe aller Nebenkomponenten und ihrer Salze liegen in Mengen von <35 %, bevorzugt <30 %, besonders bevorzugt <26 % der theoretischen Mengen vor, wobei Maleinsäure und ihre Salze (Formel 2) mit <7 %, bevorzugt <5 %, besonders bevorzugt <2 % der theoretischen Menge, Fumarsäure und ihre Salze (Formel 3) mit <20 %, bevorzugt <15 %, besonders bevorzugt <10 % der theoretischen Menge, Äpfelsäure und ihre Salze (Formel 4) mit <7 %, bevorzugt <5 %, besonders bevorzugt <3 % der theoretischen Menge und Asparaginsäure und ihre Salze (Formel 5) mit <25 %, bevorzugt <20 %, besonders bevorzugt <15 % der theoretischen Menge vorliegen.

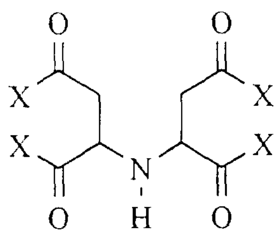

Formel 1

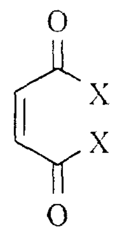

Formel 2

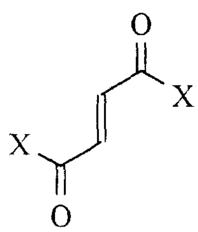

Formel 3

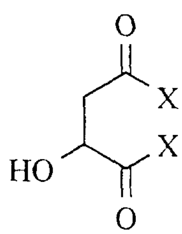

Formel 4

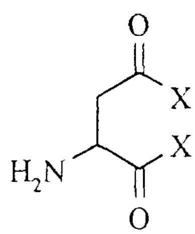

Formel 5

X = OH, OLi, ONa, OK, $ONH_4$

Insgesamt werden Produktlösungen erhalten, in denen die angegebenen Komponenten der Formeln 1-5 in Summenausbeuten von >93 %, bevorzugt >96 %, besonders bevorzugt >98 % der theoretischen Ausbeute vorliegen. Der Bioabbau der Produkte liegt entsprechend dem OECD 301 E-Test nach 28d über 70 %, meist über 72 %, vielfach über 74 %.

Die Carboxylgruppen von Iminodibernsteinsäure und ihren Nebenkomponenten liegen entsprechend der bei Reaktion und Aufarbeitung eingesetzten MeOH-Menge und der bei der Aufarbeitung abdestillierten Ammoniakmenge in Säure- oder Salzform vor. So kann Iminodibernsteinsäure für den Fall von Me=Na als $Na_2$- bis $Na_4$-Salz, bevorzugt als $Na_3$- bis $Na_4$-Salz, besonders bevorzugt als $Na_4$-Salz erhalten werden, wobei gegebenenfalls die übrigen Carboxylgruppen als freie Säure, sowie Ammoniumsalz vorliegen. Für den Fall des Einsatzes von LiOH beziehungsweise KOH oder gemischter MeOH liegen Carboxylgruppen auch als Lithium- oder Kaliumsalz vor.

Die im erfindungsgemäßen Verfahren hergestellten Produkte zeichnen sich durch sehr geringe Schwermetallgehalte aus. So liegen die Gehalte von Chrom-, Mangan-, Eisen- und Nickel-Ionen in der Summe unter 80 ppm, bevorzugt unter 60 ppm, besonders bevorzugt unter 30 ppm. Der Gehalt an Erdalkali-Ionen liegt unter 500 ppm, bevorzugt unter 200 ppm, besonders bevorzugt unter 100 ppm. Daher zeichnen sich die Produkte als wirksame Komplexiermittel für Erdalkali- und Schwermetall-Ionen aus.

In der Reaktion wird MSA in Form von Schmelze, Schuppen oder Briketts, bevorzugt Schmelze oder Schuppen, und Ammoniak flüssig, gasförmig oder im Wasser gelöst, bevorzugt flüssig oder in Wasser gelöst, eingesetzt. Wässrige Ammoniaklösungen werden mit Gehalten von >15 Gew.-%, bevorzugt >20 Gew. %, besonders bevorzugt >25 Gew. % an $NH_3$ eingesetzt. In der Reaktion und Aufarbeitung werden die Alkalimetallhydroxide MeOH in Substanz oder in wässriger Lösung verwendet. Wässrige Alkalimetallhydroxidlösungen werden in Konzentrationen von 10-60 Gew.-%, bevorzugt 20-55 Gew.-%, besonders bevorzugt 25-50 Gew.-% eindosiert.

In einer besonderen Ausführungsform wird MSA-Schmelze in wässrige Natronlauge bei Temperaturen von >60°C eindosiert und anschließend mit flüssigem Ammoniak oder mit konzentrierter wässriger Ammoniaklösung versetzt. Das MSA : NaOH : $NH_3$ : $H_2O$ Molverhältnis beträgt dabei 2:1,5-3,5:1,5-3,5:6-20. Die Edukte werden bei Temperaturen von 90-145°C und Reaktionszeiten von 0,3-25 h umgesetzt. Aus dem Umsetzungsgemisch wird unter Zusatz von Wasser und 0,5-2,5 Mol NaOH pro 2 Mol ursprünglich eingesetztem MSA Wasser und Ammoniak bei Temperaturen von 80-135°C innerhalb von 0,5-25 h abdestilliert. Nach dem Zusatz von Wasser, mit dem Feststoffgehalte von 5-60 Gew.-% eingestellt werden, und einer Klärfiltration werden Produktlösungen erhalten, die Iminodibernsteinsäure in Ausbeuten von >73 %, Maleinsäure in Mengen von <3 %, Fumarsäure in Mengen von <10 %, Äpfelsäure in Mengen von <5 % und Asparaginsäure in Mengen von <15 % der jeweiligen theoretischen Ausbeuten beziehungsweise Mengen enthalten.

In einer weiteren besonderen Ausführungsform werden MSA-Schmelze und wässrige Natronlauge simultan und kontinuierlich im Molverhältnis von MSA : NaOH : $H_2O$ = 2:1,5-3,5:6-20 in eine ebenso zusammengesetzte vorgelegte Maleinsäuresalzlösung oder pumpfähige Suspension bei Temperaturen von 75-125°C eindosiert. Mit Verweilzeiten von 0,1-5 h wird diese Lösung oder Suspension in einen zweiten Mischer gepumpt, in dem kontinuierlich flüssiges Ammoniak oder eine konzentrierte wässrige Ammoniaklösung hinzugefügt wird. Das Molverhältnis von MSA:Ammoniak beträgt dabei 2:1,5-3,5. Diese Lösung wird bei Temperaturen von 90-145°C und Verweilzeiten von 0,3-25 h in einem Konti-Reaktor umgesetzt. In einer Konti-Destillationskolonne wird aus dem Umsetzungsgemisch unter kontinuierlichem Zusatz von Wasser und wässriger Natronlauge, entsprechend 0,5-2,5 Mol NaOH pro 2 Mol ursprünglich eingesetztem MSA, Ammoniak und Wasser bei Temperaturen von 70-140°C und Verweilzeiten von 0,1-25 h abdestilliert. Nach dem Zusatz von Wasser werden Lösungen mit Feststoffgehalten von 5-60 Gew.-% erhalten, die gegebenenfalls einer Klärfiltration unterzogen werden. Die Produktlösungen weisen Ausbeuten von >73 % an Iminodibernsteinsäure, <3 % an Maleinsäure, <10 % an Fumarsäure, <5 % an Äpfelsäure und <15 % der jeweiligen theoretischen Ausbeuten an Asparaginsäure auf.

Im erfindungsgemäßen Verfahren werden durch Reaktion und Aufarbeitung Produktlösungen mit einer hohen Iminodibernsteinsäure-Ausbeute und einem hohen Komplexiervermögen erhalten. Die Nebenkomponenten beeinträchtigen weder das Komplexiervermögen noch den biologischen Abbau. Die Produkte sind weitestgehend von Ammoniak befreit. Sie sind nahezu geruchsneutral, lagerstabil und weitgehend frei von störenden Erdalkali- und Schwermetall-Ionen. Nebenkomponenten treten durch Kondensation nur in sehr untergeordnetem Maße auf und werden durch die Aufarbeitung zusätzlich veringert.

Iminodibernsteinsäurealkalisalze, insbesondere die erfindungsgemäß hergestellten, sind verwendbar zur Steigerung des Weißgrades und der Helligkeit von pflanzlichen Fasern bei der Papierherstellung, beispielsweise bei der Verarbeitung von Zellstoff oder Holzstoff (Thermo Mechanical Pulp) in der Vorbehandlung der Fasern oder in der oxidativen oder der reduktiven Bleiche, z.B. mit $H_2O_2$ bzw. mit Natriumdithionit ($Na_2S_2O_4$), insbesonders in der Vorbehandlung der Fasen vor dem Bleichprozeß oder in der reduktiven Bleiche.

## Beispiele

Die in den Beispielen angegebenen Ausbeuten und Gehalte beziehen sich entweder auf den MSA-Einsatz oder den elementaranalytisch ermittelten Kohlenstoff und wurden durch folgende Analytik erhalten: Maleinsäure, Fumarsäure und Asparaginsäure durch Flüssigkeitschromatographie (HPLC), Apfelsäure durch Kapillarelektrophorese (CE) und Iminodibernsteinsäure durch die Calciumcarbonatdispergierkapazität (CCDK).

Der CCDK-Wert wird bei pH 11 in mg $CaCO_3$ pro g Feststoff gemessen. Aus dem CCDK-Wert ergibt sich am Beispiel des Iminodibernsteinsäure $Na_4$-Salzes die Ausbeute in einer guten Näherung durch folgende Gleichung: IDS-$Na_4$-Salz [% der theoretischen Ausbeute] = (CCDK - 20):2.

Zum Vergleich ergaben sich in diesem Test für Zitronensäure-$Na_3$-Salz und Ethylendiamintetraessigsäure-$Na_4$-Salz CCDK-Werte von 55 und 280 mg $CaCO_3$ pro g Substanz. Die Durchführung des Tests verlief folgendermaßen:

1,5 g der zu untersuchenden Substanz (bei den Vergleichssubstanzen bezogen aufNa-Salz 100 %ig, bei den Produktlösungen bezogen auf den Feststoffanteil) werden in 90 ml Wasser gelöst, falls notwendig vorneutralisiert, und mit 10 ml einer 10 gew.-%igen $Na_2CO_3$-Lösung versetzt. Anschließend wird die Lösung auf pH 11 eingestellt und bei 25°C mit einer 0,10-molaren Calciumacetatlösung bis zur beginnenden Trübung, die durch ausfallendes Calciumcarbont erzeugt wird, titriert. Die Titration wird mit Hilfe eines Lichtleiterphotometers verfolgt. Aus dem ersten Knickpunkt der Titrationskurve läßt sich das verbrauchte Volumen an Calciumacetat bestimmen und daraus die von der Testsubstanz (Komplexbildner) gebundene Menge an Calcium-Ionen berechnen. Die Menge an gebundenem Calcium wird als $CaCO_3$/g Testsubstanz angegeben.

Die in den nachfolgenden Beispielen hergestellten Produktlösungen eignen sich durch die komplexierende Wirkung als Stabilisatoren für Peroxoverbindungen in wässriger Lösung. Am Beispiel von Wasserstoffperoxid wird die Stabilisierung folgendermaßen geprüft: 98 g destilliertes Wasser werden mit 1,5 g einer 33,3 %igen $H_2O_2$-Lösung versetzt. Dazu gibt man 50 mg des zu prüfenden Stabilisators (bezogen auf Na-Salz 100 %ig oder Feststoff). Anschließend wird die Mischung auf pH 10,5 eingestellt und dann für 35 Minuten bei 80°C getempert. Danach wird der $H_2O_2$-Gehalt jodometrisch bestimmt. Zum Vergleich wird der $H_2O_2$-Restgehalt in einer unter identischen Bedingungen behandelten Blindprobe (ohne Stabilisator) bestimmt. Der Stabilisierungsgrad wird dann wie folgt ermittelt:

$$((a-b) / (c-b)) \times 100 = \text{Stabilisierung [ \% ]}$$

wobei
a = $H_2O_2$-Gehalt in der stabilisierten Probe nach Temperung, b = $H_2O_2$-Gehalt in der Blindprobe nach Temperung und c = Anfangsgehalt von $H_2O_2$ in der Probe.Das in Beispiel 5 hergestellte Produkt zeigte in diesem Test eine $H_2O_2$-Stabilisierung von 96 %.

**Beispiel 1**

MSA : NaOH : $NH_3$ : $H_2O$-Molverhältnis = 2:3:2:12,6, 100°C , 48 h.
In einem 3-Liter Autoklav wurde eine Maleinsäure-Na-Salz-Lösung vorgelegt, die in der Reihenfolge aus 908 g = 50,44 Mol Wasser, 784 g = 8 Mol MSA und 480 g = 12 Mol NaOH gebildet worden war, bei 90-100°C mit 136 g = 8 Mol Ammoniak (flüssig) versetzt und bei 100°C 48 h lang gerührt. Aus dem Umsetzungsgemisch wurden nach Verdünnung mit Wasser auf 4000 g und nach Zusatz von 160 g = 4 Mol NaOH 2000 g Ammoniakwasser bei 70°C und 240 mbar abdestilliert. Die Produktlösung wurde auf 4000 g mit Wasser verdünnt und filtriert. Der für die CCDK-Wert-Messung zugrunde gelegte Feststoffgehalt betrug 33.7 Gew.-%. Folgende Ausbeuten (% der Theorie) wurden erhalten: 0,1 % Maleinsäure, 5,6 % Fumarsäure, 77,5 % Iminodibernsteinsäure und 14,6 % Asparaginsäure.

**Beispiel 2**

MSA : NaOH : $NH_3$ : $H_2O$-Molverhältnis = 2:3:2:12,6, 110°C, 12 h.
In einem 0,7-Liter Autoklav wurde eine Maleinsäure-Na-Salzlösung vorgelegt, die in der Reihenfolge aus 227 g = 12,61 Mol Wasser, 196g = 2 Mol MSA-Schuppen und 120 g = 3 Mol NaOH gebildet worden war, bei 90-100°C mit 34 g = 2 Mol Ammoniak (flüssig) versetzt und bei 110°C 12 h lang gerührt. Aus dem Umsetzungsgemisch wurden nach Verdünnung mit Wasser bei 70°C und nach Zusatz von 40 g = 1 Mol NaOH 500 g Ammoniak und Wasser bei 70°C und 240 mbar abdestilliert. Die Produktlösung wurde auf 1500 g mit Wasser verdünnt und filtiert. Der Feststoffgehalt betrug 22,5 Gew.-%. Folgende Ausbeuten (% der Theorie) wurden erhalten: 1,7 % Maleinsäure, 7,7 % Fumarsäure, 78,5 % Iminodibernsteinsäure und 11,7 % Asparaginsäure.

**Beispiel 3**

MSA : NaOH : $NH_3$ : $H_2O$-Molverhältnis = 2:3:2:12,6, 120°C, 6 h.
Die Dosierreihenfolge, die Dosiermengen und die Aufarbeitung entsprachen dem Beispiel 2. Das Reaktionsgemisch wurde bei 120°C über 6 h lang gerührt. Die Produktlösung wurde auf 1500 g mit Wasser verdünnt und filtriert Der Feststoffgehalt betrug 22,5 Gew.-%. Folgende Ausbeuten (% der Theorie) wurden erhalten: 2,0 % Maleinsäure, 8,5 % Fumarsäure, 74,5 % Iminodibernsteinsäure und 11,4 % Asparaginsäure.

**Beispiel 4**

MSA : NaOH : $NH_3$ : $H_2O$-Molverhältnis = 2:3:2:12, 120°C, 6 h
196 g = 2 Mol MSA-Schmelze und 210 g = 2,625 Mol 50%ige Natronlauge wurden simultan bei 60-70°C in eine Vorlage aus 30 g = 0,375 Mol 50 %ige Natronlauge dosiert. Nachdem bei 100°C eine klare Lösung erhalten worden war, wurden 130 g = 2 Mol 26,2 %ige wässrige Ammoniaklösung hinzugegeben, wobei eine Abkühlung auf 65°C stattfand. Die Lösung wurde anschließend in einem 0,7-Liter Autoklav bei 120°C 6 h lang gerührt. Nach Zugabe von 250 g Wasser und 80 g = 1 Mol 50%ige Natronlauge wurden Ammoniak und Wasser bei Temperaturen von 94-111°C abdestilliert.

Das Produkt wurde auf 850 g mit Wasser verdünnt und filtriert. Der Feststoffgehalt betrug 39,6 Gew.-% Folgende Ausbeuten (% der Theorie) wurden erhalten: 2,1 % Maleinsäure, 9,0 % Fumarsäure, 79,5 % Iminodibernsteinsäure und 10,1 % Asparaginsäure.

**Beispiel 5**

MSA : NaOH : $NH_3$ : $H_2O$-Molverhältnis = 2:3:2:12, 120°C, 6 h.
240 g = 3 Mol 50 %ige Natronlauge wurden vorgelegt und auf 60°C erwärmt. 196 g = 2 Mol MSA-Schmelze wurden bei 70°C eindosiert. Nachdem bei 100°C eine Lösung erhalten worden war, wurden 130 g = 2 Mol 26,2%ige wässrige Ammoniaklösung bei 60-70°C zugegeben. Die resultierende Lösung wurde in einem 0,7-Liter Autoklav bei 120°C 6 h lang gerührt. Nach Zugabe von 200 g Wasser und 80 g = 1 Mol 50%ige Natronlauge wurden 220 g Ammoniak und Wasser bei 75°C und 240 mbar abdestilliert. Das Produkt wurde mit Wasser auf 750 g verdünnt und filtriert. Der Feststoffgehalt betrug 44,9 Gew.-%. Folgende Ausbeuten (% der Theorie) wurden erhalten: 2,1 % Maleinsäure, 8,3 % Fumarsäure, 76,0 % Iminodibernsteinsäure und 11,0 % Asparaginsäure.

**Beispiel 6**

MSA : NaOH : $NH_3$ : $H_2O$-Molverhältnis = 2:3:2:12,6, 120°C, 6 h.
In eine Vorlage aus 19 kg Wasser und 306 kg = 3,825 kMol 50%ige Natronlauge wurden 250 kg = 2,55 kMol MSA-Schuppen eindosiert. Nach der Zugabe von 162 kg = 2,57 kMol 27 %iger wässriger Ammoniaklösung wurde das Gemisch bei 120°C 6 h lang gerührt. Unter Zusatz von 102,5 kg = 1,28 kMol 50 %iger Natronlauge und 755 kg Wasser wurden 450 kg Ammoniak und Wasser bei 70-80°C und 300 mbar abdestilliert. Nach der Filtration wurden 1144,5 kg Produktlösung mit einem Feststoffgehalt von 37,5 Gew.-% und Ausbeuten (% der Theorie) von 2,5 % Maleinsäure, 9,2 % Fumarsäure, 4,1% Äpfelsäure, 77,0 % Iminodibernsteinsäure und 10,0% Asparaginsäure erhalten. Das Produkt zeigte im OECD 301 E nach 28d einen biologischen Abbau von 80 %.

**Beispiel 7**

MSA : NaOH : NH3 : H2O-Molverhältnis = 2:3:2:6,7, 120°C, 3 h
In eine Vorlage aus 240 g = 3 Mol 50 %ige Natronlauge wurden 196 g = 2 Mol MSA-Schmelze bei Temperaturen >75°C eindosiert. Nach der Zugabe von 34 g = 2 Mol Ammoniak (flüssig) wurde das Reaktionsgemisch bei 120°C 3 h lang gerührt. Nach der Zugabe von 730 g Wasser und 40 g = 1 Mol NaOH wurden bei 70°C und 240 mbar 500 g Wasser und Ammoniak abdestilliert. Das Produkt wurde mit Wasser auf 1000 g verdünnt und filtriert. Der Feststoffgehalt betrug 33,7 Gew.-%. Folgende Ausbeuten (% der Theorie) wurden erhalten: 5,9 % Maleinsäure, 7,5 % Fumarsäure, 78,5 % Iminodibernsteinsäure und 8,4 % Asparaginsäure.

**Beispiel 8**

MSA : NaOH : $NH_3$ : $H_2O$-Molverhältnis = 2:3:2:6,7, 120°C, 3 h.
196 g = 2 Mol MSA wurden in 120 g = 6,7 Mol Wasser gelöst. Nach der Zugabe von 120 g = 3 Mol NaOH und 34 g = 2 Mol Ammoniak wurde das Gemisch bei 120°C 3 h gerührt. Nach der Verdünnung mit Wasser auf 1200 g und der Zugabe von 40 g = NaOH wurden 500 g Ammoniak und Wasser bei 70°C und 240 mbar andestilliert. Das Produkt wurde mit Wasser auf 1000 g verdünnt und filtriert. Der Feststoffgehalt betrug 33,7 Gew.-%. Folgende Ausbeuten (% der Theorie) wurden erhalten: 4,7 % Maleinsäure, 8,8 % Fumarsäure, 77,5 % Iminodibernsteinsäure und 8,6 % Asparaginsäure.

**Beispiel 9**

MSA : NaOH : $NH_3$: $H_2O$-Molverhältnis = 2:4:2:12,6, 100°C, 96 h.
196 g = 2 Mol MSA wurden in 227 g = 12,6 Mol Wasser gelöst. Nach der Zugabe von 160 g = 4 Mol NaOH und 34 g = 2 Mol Ammoniak wurde das Gemisch bei 100°C 96 h gerührt. Nach der Verdünnung mit Wasser auf 1200 g wurden 500 g Ammoniak und Wasser bei 70°C und 240 mbar abdestilliert. Das Produkt wurde mit Wasser auf 1500 g verdünnt und filtriert. Der Feststoffgehalt betrug 22,5 Gew.-%.

Folgende Ausbeuten (% der Theorie) wurden erhalten: 2,7 % Maleinsäure, 5,6 % Fumarsäure, 80,5 % Iminodibernsteinsäure und 7,3 % Asparaginsäure.

**Beispiel 10**

MSA : NaOH : $NH_3$ : $H_2O$-Molverhältnis = 2:2:2:12,6, 110°C, 12 h.
196 g = 2 Mol MSA wurden in 227 g = 12,6 Mol Wasser gelöst. Nach der Zugabe von 80 g = 2 Mol NaOH und 34 g = 2 Mol Ammoniak wurde das Gemisch bei 110°C 12 h gerührt. Nach der Verdünnung mit Wasser auf 1200 g und der Zugabe von 80 g = 2 Mol NaOH wurden 500 g Ammoniak und Wasser bei 70°C und 240 mbar abdestilliert. Das Produkt wurde mit Wasser auf 1000 g verdünnt und filtriert. Der Feststoffgehalt betrug 33,7 Gew.-%. Folgende Ausbeuten (% der Theorie) wurden erhalten: 0,5 % Maleinsäure, 6,7 % Fumarsäure, 77,0 % Iminodibernsteinsäure und 11,8 % Asparaginsäure.

**Beispiel 11**

MSA : NaOH : $NH_3$ : $H_2O$-Molverhältnis = 2:2:2:12,6, 120°C, 3 h.
196 g = 2 Mol MSA wurden in 227 g = 12,6 Mol Wasser gelöst. Nach der Zugabe von 80 g = 2 Mol NaOH und 34 g = 2 Mol Ammoniak wurde das Gemisch bei 120°C 3 h gerührt. Nach der Verdünnung mit Wasser auf 1200 g und der Zugabe von 80 g = 2 Mol NaOH wurden 500 g Ammoniak und Wasser bei 70°C und 240 mbar abdestilliert. Das Produkt wurde mit Wasser auf 1000 g verdünnt und filtriert. Der Feststoffgehalt betrug 33,7 Gew.-%. Folgende Ausbeuten (% der Theorie) wurden erhalten: 2,7 % Maleinsäure, 7,2 % Fumarsäure, 75,0 % Iminodibernsteinsäure und 11,9 % Asparaginsäure.

**Beispiel 12**

MSA : NaOH : $NH_3$ : $H_2O$-Molverhältnis = 2:2:3:12,6, 100°C, 24 h.
196 g = 2 Mol MSA wurden in 227 g = 12,6 Mol Wasser gelöst. Nach der Zugabe von 80 g = 2 Mol NaOH und 51 g = 3 Mol Ammoniak wurde das Gemisch bei 100°C 24 h gerührt. Nach der Verdünnung mit Wasser auf 1200 g und der Zugabe von 80g = 2 Mol NaOH wurden 500 g Ammoniak und Wasser bei 70°C und 240 mbar abdestilliert. Das Produkt wurde mit Wasser auf 1500 g verdünnt und filtriert. Der Feststoffgehalt betrug 22,5 Gew.-%. Folgende Ausbeuten (% der Theorie) wurden erhalten: 0,6 % Maleinsäure, 4,5 % Fumarsäure, 79,5 % Iminodibernsteinsäure und 15,4 % Asparaginsäure.

**Beispiel 13**

MSA : NaOH : $NH_3$ : $H_2O$-Molverhältnis = 2:2:4:12,6, 100°C, 12 h.
196 g = 2 Mol MSA wurden in 227 g = 12,6 Mol Wasser gelöst. Nach der Zugabe von 80 g = 2 Mol NaOH und 68 g = 3 Mol Ammoniak wurde das Gemisch bei 100°C 12 h gerührt. Nach der Verdünnung mit Wasser auf 1200 g und der Zugabe von 80 g = 2 Mol NaOH wurden 500 g Ammoniak und Wasser bei 70°C und 240 mbar abdestilliert. Das Produkt wurde mit Wasser auf 1500 g verdünnt und filtriert. Der Feststoffgehalt betrug 22,5 Gew.-%. Folgende Ausbeuten (% der Theorie) wurden erhalten: 3,4 % Maleinsäure, 4,5 % Fumarsäure, 76,5 % Iminodibernsteinsäure und 14,5 % Asparaginsäure.

**Beispiel 14**

MSA : NaOH : $NH_3$ : $H_2O$-Molverhältnis = 2:2:2:10, 120°C, 3,5 h.
In eine Vorlage aus 180 g = 10 Mol Wasser und 80 g = 2 Mol NaOH wurden in einen Autoklav 196 g = 2 Mol MSA-Schmelze bei Temperaturen von >75°C gepumpt Nach der Zugabe von 34 g = 2 Mol Ammoniak wurde das Gemisch bei 120°C 3,5 h lang gerührt. Das Umsetzungsgemisch wurde mit 200 g Wasser und 80 g = 2 Mol NaOH versetzt, und ca. 130 g Ammoniak und Wasser wurden abdestilliert. Dabei stieg die Temperatur an. Bei 110°C wurden unter Normaldruck innerhalb von 1 h 100 g Wasser hinzugesetzt und wiederum abdestilliert. Das nahezu geruchsfreie Produkt wurde mit Wasser auf 800 g verdünnt und filtriert. Der Feststoffgehalt betrug 42,1 Gew.-%. Folgende Ausbeuten (% der Theorie) wurden erhalten: 0,6 % Maleinsäure, 6,7 % Fumarsäure, 75,5 % Iminodibernsteinsäure und 13,3 % Asparaginsäure.

**Beispiel 15**

MSA : NaOH : $NH_3$ : $H_2O$-Molverhältnis = 2:2:2:10, 110°C, 7 h.
In eine Vorlage aus 180 g = 10 Mol Wasser und 80 g = 2 Mol NaOH wurden in einen Autoklav 196 g = 2 Mol MSA-Schmelze bei Temperaturen von >75°C gepumpt Nach der Zugabe von 34g = 2 Mol Ammoniak wurde das Gemisch bei 110°C 7 h lang gerührt. Das Umsetzungsgemisch wurde mit 200 g Wasser und 80 g = 2 Mol NaOH versetzt, und ca. 130 g Ammoniak und Wasser wurden abdestilliert. Dabei stieg die Temperatur an. Bei 110°C wurden unter Normaldruck

innerhalb von 1 h 100 g Wasser hinzugesetzt und wiederum abdestilliert. Das nahezu geruchsfreie Produkt wurde mit Wasser auf 800 g verdünnt und filtriert. Der Feststoffgehalt betrug 42,1 Gew.-%. Folgende Ausbeuten (% der Theorie) wurden erhalten: 1,3 % Maleinsäure, 5,9 % Fumarsäure, 80,0 % Iminodibernsteinsäure und 11,2 % Asparaginsäure.

**Beispiel 16**

MSA : NaOH : $NH_3$ : $H_2O$-Molverhältnis = 2:2:2.10, 130°C, 1,5 h.
In eine Vorlage aus 180 g = 10 Mol Wasser und 80 g = 2 Mol NaOH wurden in einen Autoklav 196 g = 2 Mol MSA-Schmelze bei Temperaturen von >75°C gepumpt. Nach der Zugabe von 34 g = 2 Mol Ammoniak wurde das Gemisch bei 130°C 1,5 h lang gerührt. Das Umsetzungsgemisch wurde mit 200 g Wasser und 80 g = 2 Mol NaOH versetzt, und ca. 130 g Ammoniak und Wasser wurden abdestilliert. Dabei steigt die Temperatur an. Bei 110°C wurden unter Normaldruck innerhalb von 1 h 100 g Wasser hinzugesetzt und wiederum abdestilliert. Das nahezu geruchsfreie Produkt wurde mit Wasser auf 800 g verdünnt und filtriert. Der Feststoffgehalt betrug 42,1 Gew.-%. Folgende Ausbeuten (% der Theorie) wurden erhalten: 0,6 % Maleinsäure, 6,5 % Fumarsäure, 75,0 % Iminodibernsteinsäure und 15,6 % Asparaginsäure.

**Beispiel 17**

In einen 1-Liter Rührkesselreaktor wurden in eine vorgelegte Maleinsäure-Na-Salzlösung der herzustellenden Zusammensetzung simultan und kontinuierlich 355 g/h = 3,62 Mol/h MSA und 501 g/h = 3,86 Mol/h 30,8 Gew.-%ige Natronlauge unter Kühlung bei einer Temperatur von 118°C eindosiert. Die entstehende Lösung wurde kontinuierlich in eine aus drei Reaktoren mit einem Gesamtvolumen von 7,4 Litern bestehende Rührkesselkaskade gepumpt. Im ersten Reaktor der Kaskade wurden 68 g/h = 4 Mol/h Ammoniak gasförmig zugemischt. Die Temperatur der Reaktionslösung wurde auf 109-114°C gehalten. Anschließend wurde das Umsetzungsprodukt in einem statischen Mischer kontinuierlich mit 991 g/h = 3,92 Mol/h 15,8 Gew.-%iger Natronlauge vermischt. Danach wurde die Lösung auf den Kopf einer aus zehn Böden bestehenden und mit ca. 520 g/h Strippdampf betriebenen Glockenbodenkolonne gefahren. Bei einer Sumpftemperatur von 112°C , einer Kopftemperatur von 101°C und einem Flüssigkeitsvolumen von ca. 1,3 Liter wurden 1037 g/h Ammoniak und Wasser abdestilliert und 1398 g/h an Produktlösung erhalten. Der Feststoffgehalt betrug 43,8 Gew.-%. Folgende Ausbeuten (% der Theorie) wurden erhalten. 2,3 % Maleinsäure, 9,2 % Fumarsäure, 77,0 % Iminodibernsteinsäure und 11,5 % Asparginsäure.

**Verwendungsbeispiel 1**

Erhöhung von Weißgrad und Helligkeit bei der oxidativen Bleiche von Holzstoff (Thermo-Mechanical-Pulp) durch den Einsatz des Produktes aus Beispiel 6 in der Vorbehandlung.

Der Holzstoff wurde bei einer Stoffdichte von 4 % im Desintegrator bei 3000 U/min über 10 min aufgeschlagen. Zu der gerührten Suspension wurde das Produkt aus Beispiel 6 als 1,3 Gew.-%ige Lösung, bezogen auf Feststoff, gegeben. Die Feststoff-Menge (= Summe aller Na-Salze) entsprach dabei 0,13-0,52%, bezogen auf ofentrockenen (otro) Faserstoff. Die Einwirkzeit betrug 30 min bei einer Temperatur von 80°C. Mittels einer Laborfilterpresse erfolgte die Entwässerung auf die für den Bleichvorgang benötigte Stoffdichte von 25 %. Dadurch wurde ein Teil der komplexierten Schwermetallionen über das Filtrat von dem Faserstoff abgetrennt. Zu dem resultierenden Faserstoff wurden 0,7 % NaOH als 1 Gew.-%ige Lösung und 2 % Wasserstoffperoxid als 20 Gew.-%ige Lösung, bezogen auf den otro Faserstoff, gegeben. Mittels eines Labormixers wurde eine intensive Durchmischung und gleichmäßige Verteilung erreicht. Die Bleichdauer betrug 2,5 h bei 60°C. Nach der Bleiche wurden mit einem Rapid-Köthen-Blattbildner Problätter hergestellt und Weißgrad und Helligkeit nach DIN 53.145 beziehungsweise 53.140 bestimmt. Zum Vergleich wurde ein Versuch ohne die Zugabe eines Komplexiermittels in der Vorbehandlung durchgeführt. Folgende Ergebnisse wurden erhalten.

| Zugabe von Produkt aus Beispiel 6; Feststoff [%], bez. auf otro Faserstoff | Weißgrad [%] | Helligkeit [%] |
|---|---|---|
| 0,00 | 57,4 | 71,2 |
| 0,13 | 57,7 | 72,3 |
| 0,26 | 58,8 | 73,6 |
| 0,39 | 60,5 | 75,3 |
| 0,52 | 61,9 | 76,6 |

In Gegenwart des Produktes aus Beispiel 6 ist eine deutliche Erhöhung von Weißgrad und Helligkeit festzustellen.

**Verwendungsbeispiel 2**

Erhöhung von Weißgrad und Helligkeit bei der oxidativen Bleiche von Holzstoff (Thermo-Mechanical-Pulp) durch den Einsatz des Produktes aus Beispiel 6:

Der Holzstoff wurde wie im Verwendungsbeispiel 1 bei einer Stoffdichte von 4 % im Desintegrator bei 3000 U/min aufgeschlagen. Nach einer Einwirkzeit von 30 min bei 80°C wurde die Suspension mittels einer Laborfilterpresse auf eine Stoffdichte von 25 % aufkonzentriert. Zu dem resultierenden Faserstoff wurden 0,7 % NaOH als 1 %ige Lösung, 2 % Wasserstoffperoxid als 20 %ige Lösung und 0,13-0,52 % Produkt (entspricht dem Feststoff) aus Beispiel 6 als 1,3 %ige Lösung gegeben. Der jeweilige Mengeneinsatz bezieht sich auf den otro Faserstoff. Mittels eines Labormixers wurde eine intensive Durchmischung und gleichmäßige Verteilung erreicht. Die Bleichdauer betrug 2,5 h bei 60°C. Nach der Bleiche wurden mit einem Rapid-Köthen-Blattbildner Probeblätter hergestellt und Weißgrad und Helligkeit nach DIN 53.145 beziehungsweise 53.140 bestimmt. Zum Vergleich wurde ein Bleichversuch ohne Komplexiermittel durchgeführt. Folgende Ergebnisse wurden erhalten:

| Zugabe von Produkt aus Beispiel 6; Feststoff [%], bez. auf otro Faserstoff | Weißgrad [%] | Helligkeit [%] |
|---|---|---|
| 0,00 | 57,4 | 71,2 |
| 0,13 | 58,7 | 72,2 |
| 0,26 | 59,5 | 73,2 |
| 0,39 | 60,4 | 74,3 |
| 0,52 | 61,0 | 75,0 |

In Gegenwart des Produktes aus Beispiel 6 ist eine deutliche Erhöhung von Weißgrad und Helligkeit festzustellen.

**Verwendungsbeispiel 3**

Erhöhung des Weißgrades bei der reduktiven Bleiche von Holzstoff (Thermo-Mechanical-Pulp) durch den Einsatz des Produktes aus Beispiel 6:

Der Holzstoff wurde bei einer Stoffdichte von 4 % bei 60°C mit 0,26-0,52 % Produkt (entspricht dem Feststoff) aus Beispiel 6 und unter Luftausschluß mit 1,0-1,5 % Natriumdithionit techn. (ca. 85 %ig) versetzt. Der jeweilige Mengeneinsatz bezieht sich auf den otro Faserstoff. Nach einer Bleichdauer von 1h bei 60°C (Beutelbleiche) und einem pH-Wert von 6,0 wurde der Weißgrad des TMP bestimmt. Zum Vergleich wurde ein Bleichversuch ohne Komplexiermittel durchgeführt. Folgende Ergebnisse wurden erhalten:

| Zugabe von Produkt aus Beispiel 6; Feststoff [%], bez. auf otro Faserstoff | Weißgrad [%] bei 1,0 % $Na_2S_2O_4$ | Weißgrad [%] bei 1,5 % $Na_2S_2O_4$ |
|---|---|---|
| 0,00 | 52,3 | 53,1 |
| 0,26 | 53,6 | 54,3 |
| 0,39 | 54,0 | 54,1 |
| 0,52 | 53,8 | 53,9 |

In Gegenwart des Produktes aus Beispiel 6 ist eine Erhöhung des Weißgrades festzustellen.

**Patentansprüche**

1. Verfahren zur Herstellung von Iminodibernsteinsäurealkalisalzen, **dadurch gekennzeichnet, dass** Maleinsäureanhydrid (MSA), Alkalimetallhydroxid (MeOH), Ammoniak ($NH_3$) und Wasser im Molverhältnis von MSA : MeOH : $NH_3$ : $H_2O$ = 2:0,1-4:1,1-6:5-30 bei Temperaturen von 70-170°C, unter Drücken von 1-80 bar und Reaktionszeiten von 0,1-100 h umgesetzt werden, aus dem Umsetzungsgemisch unter Zusatz von Wasser und 0-4 Mol MeOH pro 2 Mol ursprünglich eingesetztem MSA Ammoniak und Wasser bei Temperaturen von 50-170°C unter Drücken von

0,1-50 bar innnerhalb von 0,1-50 h abdestilliert werden und nach der Destillation Wasser in einer solchen Menge zugesetzt wird, dass die entstehende Lösung einen Feststoffgehalt von 5-60 %, bezogen auf das Gesamtgewicht der Lösung, enthält.

**2.** Verfahren nach Anspruch 1, **dadurch gekennzeichnet, dass** MSA, MeOH, Ammoniak und Wasser bei Temperaturen von 80-160, bevorzugt bei 85-150, besonders bevorzugt bei 90-145°C, umgesetzt werden.

**3.** Verfahren nach Ansprüchen 1 und 2, **dadurch gekennzeichnet, dass** nach der Reaktion Ammoniak und Wasser unter Zusatz von Wasser und 0,5-3,5 Mol, bevorzugt 0,7-3,0 Mol, besonders bevorzugt 0,9-2,5 Mol MeOH pro 2 Mol ursprünglich eingesetztem MSA, abdestilliert werden.

**4.** Verfahren nach Ansprüchen 1 bis 3, **dadurch gekennzeichnet, dass** nach der Reaktion Ammoniak und Wasser bei Temperaturen von 60-150°C, bevorzugt 70-140°C, besonders bevorzugt 80-135°C und Drücken von 0,5-20 bar innerhalb von 0,3-30 h, bevorzugt 0,5-25 h, besonders bevorzugt 0,9-20 h, abdestilliert werden.

**5.** Verfahren nach Ansprüchen 1 bis 4, **dadurch gekennzeichnet, dass** nach der Destillation von Ammoniak und Wasser die Produktlösung durch Zugabe von Wasser auf Feststoffgehalte, gerechnet als Summe aller Alkalisalze, von 10-58 Gew.-%, bevorzugt 15-55 Gew.-%, besonders bevorzugt 20-50 Gew.-% eingestellt und gegebenenfalls einer Klärfiltration unterzogen wird.

**6.** Verfahren nach Ansprüchen 1 bis 5, **dadurch gekennzeichnet, dass** NaOH in einer solchen Menge eingesetzt wird, dass Iminodibernsteinsäure-$(Na)_2$ bis -$(Na)_4$-Salze, bevorzugt $(Na)_3$- bis $(Na)_4$-Salze, besonders bevorzugt $(Na)_4$-Salze, erhalten werden, wobei gegebenenfalls die übrigen Carboxylgruppen als freie Säure sowie als Lithium-, Kalium- oder Ammonium-Salz vorliegen.

**7.** Verfahren nach Ansprüchen 1 bis 6, **dadurch gekennzeichnet, dass** die Verfahrensschritte Reaktion und Aufarbeitung sowie die dazugehörigen Dosierungen der Komponenten sowohl diskontinuierlich als auch kontinuierlich durchgeführt werden können.

**8.** Verfahren nach Ansprüchen 1 bis 7 **dadurch gekennzeichnet, dass** der Reihenfolge nach wässrige Natronlauge, MSA und Ammoniak oder eine wässrige Ammoniak-Lösung im Molverhältnis von MSA : NaOH : $NH_3$ : Wasser = 2:1,5-3,5:1,5-3,5:6-20 in einen Reaktor dosiert werden, bei Temperaturen von 90-145°C und Reaktionszeiten von 0,3-25 h umgesetzt werden, unter Zugabe von Wasser und 0,5-2,5 Mol NaOH pro Mol ursprünglich eingesetztem MSA Ammoniak und Wasser bei Temperaturen von 80-135°C innerhalb von 0,5-25 h abdestilliert werden und nach der Destillation Wasser in einer solchen Menge zugesetzt wird, dass die entstehende Lösung einen Feststoffgehalt von 5 bis 60 %, bezogen auf das Gesamtgewicht der Lösung, enthält.

**9.** Verfahren nach Ansprüchen 1 bis 7, **dadurch gekennzeichnet, dass** MSA und wässrige Natronlauge simultan und kontinuierlich im Molverhältnis von MSA : NaOH : $H_2O$ = 2:1,5-3,5:6-20 in eine entsprechend zusammengesetzte vorgelegte Maleinsäuresalzlösung eindosiert werden, diese Lösung nach Verweilzeiten von 0,1-5 h in einem separaten Mischer kontinuierlich mit Ammoniak oder wässriger Ammoniaklösung in einem Molverhältnis von MSA: $NH_3$ = 2:1,5-3,5 versetzt wird, die resultierende Lösung unter Verweilzeiten von 0,3-25 h bei Temperaturen von 90-145°C in einem Konti-reaktor umgesetzt werden, aus dem Umsetzungsgemisch kontinuierlich Ammoniak und Wasser unter kontinuierlichem Zusatz von Wasser und 0,5-2,5 Mol NaOH pro 2 Mol ursprünglich eingesetztem MSA abdestilliert werden und nach der Destillation Wasser kontinuierlich in einer solchen Menge zugesetzt wird, dass die entstehende Lösung einen Feststoffgehalt von 5-60%, bezogen auf das Gesamtgewicht der Lösung, enthält.

## Claims

**1.** Process for the preparation of iminodisuccinic acid alkali metal salts, **characterized in that** maleic anhydride (MA), alkali metal hydroxide (MeOH), ammonia ($NH_3$) and water are reacted in a molar ratio of MA : MeOH : $NH_3$ : $H_2O$ = 2:0.1-4:1.1-6:5-30 at temperatures of 70-170°C, under pressures of 1-80 bar over reaction times of 0.1-100 h, ammonia and water are distilled off from the reaction mixture at temperatures of 50-170°C under pressures of 0.1-50 bar in the course of 0.1-50 h, with the addition of water and 0-4 mol of MeOH per 2 mol of MA originally employed, and after the distillation water is added in an amount such that the solution formed contains a solids content of 5-60%, based on the total weight of the solution.

2. Process according to Claim 1, **characterized in that** MA, MeOH, ammonia and water are reacted at temperatures of 80-160, preferably 85-150, particularly preferably 90-145°C.

3. Process according to Claims 1 and 2, **characterized in that**, after the reaction, ammonia and water are distilled off with the addition of water and 0.5-3.5 mol, preferably 0.7-3.0 mol, particularly preferably 0.9-2.5 mol of MeOH per 2 mol of MA originally employed.

4. Process according to Claims 1 to 3, **characterized in that**, after the reaction, ammonia and water are distilled off at temperatures of 60-150°C, preferably 70-140°C, particularly preferably 80-135°C, under pressures of 0.5-20 bar in the course of 0.3-30 h, preferably 0.5-25 h, particularly preferably 0.9-20 h.

5. Process according to Claims 1 to 4, **characterized in that**, after the distillation of ammonia and water, the product solution is adjusted to solids contents, calculated as the sum of all the alkali metal salts, of 10-58% by weight, preferably 15-55% by weight, particularly preferably 20-50% by weight, by addition of water and, if appropriate, is subjected to a clarifying filtration.

6. Process according to Claims 1 to 5, **characterized in that** NaOH is employed in an amount such that iminodisuccinic acid $(Na)_2$ to $(Na)_4$ salts, preferably $(Na)_3$ to $(Na)_4$ salts, particularly preferably $(Na)_4$ salts, are obtained, the other carboxyl groups being present, if appropriate, as the free acid and as the lithium, potassium or ammonium salt.

7. Process according to Claims 1 to 6, **characterized in that** the process steps of reaction and working up and the associated meterings of the components can be carried out both discontinuously and continuously.

8. Process according to Claims 1 to 7, **characterized in that** aqueous sodium hydroxide solution, MA and ammonia or an aqueous ammonia solution in a molar ratio of MA : NaOH : $NH_3$ : water = 2:1.5-3.5:1.5-3.5:6-20 are metered in sequence into a reactor and are reacted at temperatures of 90-145°C with reaction times of 0.3-25 h, ammonia and water are distilled off at temperatures of 80-135°C in the course of 0.5-25 h, with the addition of water and 0.5-2.5 mol of NaOH per mol of MA originally employed, and, after the distillation, water is added in an amount such that the solution formed contains a solids content of 5 to 60%, based on the total weight of the solution.

9. Process according to Claims 1 to 7, **characterized in that** MA and aqueous sodium hydroxide solution are metered simultaneously and continuously in a molar ratio of MA : NaOH : $H_2O$ = 2:1.5-3.5:6-20 into an initially introduced maleic acid salt solution of corresponding composition, after residence times of 0.1-5 h ammonia or aqueous ammonia solution is added to this solution in a separate mixer in a molar ratio of MA : $NH_3$ = 2:1.5-3.5, the resulting solution is reacted in a continuous reactor under residence times of 0.3-25 h at temperatures of 90-145°C, ammonia and water are distilled off continuously from the reaction mixture, with continuous addition of water and 0.5-2.5 mol of NaOH per 2 mol of MA originally employed, and, after the distillation, water is added continuously in an amount such that the resulting solution contains a solids content of 5-60%, based on the total weight of the solution.

## Revendications

1. Procédé pour la préparation de sels alcalins de l'acide iminodisuccinique, **caractérisé en ce que** l'on fait réagir l'anhydride maléique (AM), un hydroxyde de métal alcalin (MeOH), l'ammoniac ($NH_3$) et de l'eau dans des proportions molaires AM/MeOH/$NH_3$/$H_2O$ de 2:0,1 à 4:1,1 à 6:5 à 30 à des températures de 70 à 170°C sous des pressions de 1 à 80 bars et dans des durées de réaction de 0,1 à 100 h, on distille du mélange de réaction, en ajoutant de l'eau et 0 à 4 mol de MeOH pour 2 mol d'AM mis en oeuvre à l'origine, de l'ammoniac et de l'eau à des températures de 50 à 170°C sous des pressions de 0,1 à 50 bars en 0,1 à 50 h et après la distillation, on ajoute de l'eau en quantité telle que la solution obtenue ait une teneur en matières solides de 5 à 60 % de son poids total.

2. Procédé selon la revendication 1, **caractérisé en ce que** l'AM, le MeOH, l'ammoniac et l'eau sont mis à réagir à des températures de 80 à 160, de préférence de 85 à 150 et plus spécialement de 90 à 145°C.

3. Procédé selon les revendications 1 et 2, **caractérisé en ce que**, après la réaction, on distille l'ammoniac et l'eau en ajoutant de l'eau et 0,5 à 3,5 mol, de préférence 0,7 à 3,0 mol et plus spécialement 0,9 à 2,5 mol de MeOH pour 2 mol d'AM mis en oeuvre à l'origine.

4. Procédé selon les revendications 1 à 3, **caractérisé en ce que**, après la réaction, on distille l'ammoniac et l'eau à des températures de 60 à 150°C, de préférence de 70 à 140°C et plus spécialement de 80 à 135°C sous des pressions de 0,5 à 20 bars dans des durées de 0,3 à 30 h, de préférence de 0,5 à 25 h et plus spécialement de 0,9 à 20 h.

5. Procédé selon les revendications 1 à 4, **caractérisé en ce que**, après distillation de l'ammoniac et de l'eau, on règle la solution de produit, par addition d'eau, à des teneurs en matières solides, exprimées par la somme de tous les sels alcalins, de 10 à 58 % en poids, de préférence de 15 à 55 % en poids et plus spécialement de 20 à 50 % en poids et le cas échéant, on soumet à une clarification.

6. Procédé selon les revendications 1 à 5, **caractérisé en ce que** le NaOH est mis en oeuvre en quantité telle que l'on obtienne les sels de $Na_2$ à $Na_4$ de l'acide iminodisuccinique, de préférence les sels de $Na_3$ à $Na_4$ et plus spécialement les sels de $Na_4$, les autres groupes carboxyle éventuels étant à l'état d'acide libre ou de sels de lithium, de potassium ou d'ammonium.

7. Procédé selon les revendications 1 à 6, **caractérisé en ce que** les divers stades opératoires de réaction et de traitements consécutifs et les dosages correspondants des composants peuvent être mis en oeuvre en discontinu ou en continu.

8. Procédé selon les revendications 1 à 7, **caractérisé en ce que** l'on introduit dans un réacteur, dans l'ordre indiqué, la lessive de soude, l'AM et l'ammoniac ou l'ammoniaque à des proportions molaires AM/NaOH/$NH_3$/eau = 2:1,5 à 3,5:1,5 à 3,5:6 à 20, on les fait réagir à des températures de 90 à 145°C dans des durées de réaction de 0,1 à 25 h, on distille l'ammoniac et l'eau à des températures de 80 à 135°C en une durée de 0,5 à 25 h avec adjonction d'eau et de 0,5 à 2,5 mol de NaOH/mol d'AM mis en oeuvre à l'origine et après la distillation, on ajoute de l'eau en quantité telle que la solution obtenue ait une teneur en matières solides de 5 à 60 % de son poids total.

9. Procédé selon les revendications 1 à 7, **caractérisé en ce que** l'on introduit l'AM et la lessive de soude simultanément et en continu à des proportions molaires AM/NaOH/$H_2O$ = 2:1,5 à 3,5:6 à 20 dans une solution de sel de l'acide maléique à la composition correspondante, on ajoute à cette solution, après des durées de contact de 0,1 à 5 h, dans un mélangeur séparé, en continu, de l'ammoniac ou de l'ammoniaque à un rapport molaire AM/$NH_3$ = 2:1,5 à 3,5, on fait réagir la solution obtenue dans des durées de contact de 0,3 à 25 h à des températures de 90 à 145°C dans un réacteur continu, on distille en continu du mélange de réaction de l'ammoniac et de l'eau avec addition continue d'eau et de 0,5 à 2,5 mol de NaOH pour 2 mol d'AM mis en oeuvre à l'origine, et après cette distillation, on ajoute de l'eau en continu, en quantité telle que la solution formée ait une teneur en matières solides de 5 à 60 % de son poids total.